# EUROPEAN PATENT SPECIFICATION

(11) **EP 3 172 571 B1**
(45) Date of publication and mention of the grant of the patent: **11.12.2019**
(21) Application number: 15747518.7
(22) Date of filing: 22.07.2015
(51) Int. Cl.: G01N 33/68

(54) **MARKERS FOR ATRIAL FIBRILLATION (AF)**
VORHOFFLIMMERN - MARKERS
MARQUEURS POUR LA FIBRILLATION AURICULAIRE (AF)

(30) Priority: 23.07.2014 GB 201413081
(43) Date of publication of application: 31.05.2017
(73) Proprietor: The University Of Birmingham, Birmingham B15 2TT (GB)
(72) Inventor: FABRITZ, Larissa, Birmingham West Midlands B15 2TT (GB); KIRCHHOF, Paulus, Birmingham West Midlands B15 2TT (GB)
(74) Representative: Chapman, Paul Gilmour
(86) International application number: PCT/GB2015/052112
(87) International publication number: WO 2016/012783

(56) References cited:
- WO-A1-2014/072500
- MILENA DAMJANAC ET AL: "PKR, a cognitive decline biomarker, can regulate translation via two consecutive molecular targets p53 and Redd1 in lymphocytes of AD patients", JOURNAL OF CELLULAR AND MOLECULAR MEDICINE, vol. 13, no. 8b, 4 February 2009 (2009-02-04), pages 1823-1832, XP55096928, ISSN: 1582-1838, DOI: 10.1111/j.1582-4934.2009.00688.x
- PETER C. KAHR ET AL: "Systematic Analysis of Gene Expression Differences between Left and Right Atria in Different Mouse Strains and in Human Atrial Tissue", PLOS ONE, vol. 6, no. 10, 19 October 2011 (2011-10-19), page e26389, XP55188268, DOI: 10.1371/journal.pone.0026389
- ARNE YNDESTAD ET AL: "The Homeostatic Chemokine CCL21 Predicts Mortality and May Play a Pathogenic Role in Heart Failure", PLOS ONE, vol. 7, no. 3, 12 March 2012 (2012-03-12), page e33038, XP55214639, DOI: 10.1371/journal.pone.0033038
- STEINBERG BENJAMIN A ET AL: "Use and outcomes of antiarrhythmic therapy in patients with atrial fibrillation receiving oral anticoagulation: Results from the ROCKET AF trial", HEART RHYTHM, vol. 11, no. 6, June 2014 (2014-06), pages 925-932, XP029027235, ISSN: 1547-5271, DOI: 10.1016/J.HRTHM.2014.03.006
- C. E. WOODS ET AL: "Atrial Fibrillation Therapy Now and in the Future: Drugs, Biologicals, and Ablation", CIRCULATION RESEARCH, vol. 114, no. 9, 24 April 2014 (2014-04-24), pages 1532-1546, XP055214740, ISSN: 0009-7330, DOI: 10.1161/CIRCRESAHA.114.302362

## Description

### Field of the Invention

The present invention relates to methods of detecting one or more markers in a sample obtained from a subject in order to ascertain whether or not the subject may be predisposed to developing and/or is suffering from atrial fibrillation (AF), such as paroxysmal AF.

### Background to the Invention

Atrial fibrillation (AF) is a common cause of stroke, death, heart failure, and hospitalizations in Europe and in the world. However, while AF affects 1-2% of the population, it is often not detected prior to a first complication (often a stroke). Moreover, although chronic forms of AF can be picked up with a simple ECG, paroxysmal, self-terminating forms of AF are notoriously difficult to detect (e.g. false negative rates of 50-70% even when 12 days of Holter ECG monitoring are applied over a year, Kirchhof P, et al. Eur Heart J. 30:2969-2977c (2009)). Paroxysmal AF causes up to 15% of strokes in unselected cohorts of stroke survivors (e.g. Grond, et al. Stroke. 44:3357-3364 (2013); Sanna, et al. N Engl J Med. 370:2478-2486 (2014))). Timely diagnosis of AF and initiation of anticoagulation would dramatically help to prevent ischemic strokes.

A screening test for paroxysmal AF would save the health care system billions of pounds by preventing strokes via timely initiation of oral anticoagulation (Kirchhof P, et al. Eur Heart J. 34:1471-1474 (2013)). As the implantation of a subcutaneous ECG recorder, currently the "gold standard" for the detection of paroxysmal AF (Sanna T, et al. N Engl J Med. 370:2478-2486 (2014), is costly (ca £3000) and an invasive procedure with a 1-2% risk for the patient, simpler and/or better tests to identify patients with paroxysmal AF are urgently needed.

Common gene variants close to the pitx2 gene are found in patients with AF. It has long been known that AF has a strong familial component, especially in younger patients. Recent genome-wide association studies identified at least three independent common gene variants close on chromosome 4q25 that are strikingly associated with fibrillation (Ellinor PT, et al. Nat Genet. 44:670-675 (2012); Gudbjartsson DF, et al. Nature. 448:353-357 (2007)). The gene that is closest to these variants codes for the bidomain transcription factor PITX2. While the role of PITX2 in the prenatal development of heart and lung has been well characterised, the physiological role of PITX2 in the adult heart was not known until recently.

PITX2 is expressed in the left atrium and reducing pitx2 predisposes a subject to AF. It has been demonstrated that pitx2 is present in the adult left atrium, and that reducing the level of pitx2 in the heart predisposes subjects to AF (Kirchhof P, et al. Circ Cardiovasc Genet. 4:123-133 (2011)). The development of AF in atria with reduced PITX2 expression was confirmed by others (Wang J, et al. Proc Natl Acad Sci USA. 107:9753-9758 (2010); Chinchilla A, et al. Circ Cardiovasc Genet. 4:269-279 (2011)). One important and surprising finding of this background research is the limitation of PITX2 expression to left atrial tissue, suggesting that PITX2 has a physiological role in maintaining normal left atrial gene expression.

Kahr PC, et al. (PLoS ONE. 6:e26389 (2011)) discloses the results of systematic analysis of gene expression differences using gene arrays between left and right atria from mouse and human atrial tissue. 10 genes were identified as being differentially expressed in left atrium and half of these genes show reduced expression in left atrial tissue from mice with heterozygous deletion of pitx2, strongly suggesting regulation of their expression by pitx2. The authors speculate that expression of ccl21 might be associated with development of AF based on an indirect comparison of gene arrays taken from mice with normal and reduced pitx2 expression. However, several important measurements are lacking, namely proper confirmation that any of these genes are actually expressed in a pitx2-dependent fashion in the left atrium of mice (e.g. by rtPCR), confirmation that reduced expression levels of any of the identified genes are found in atrial tissue of patients with atrial fibrillation, a documentation that reduced expression of the aforementioned genes actually results in reduced expression of the gene product, and finally a suggestion how to measure this in patients, where access to the left atrium is limited. In addition, as ccl21 is expressed in the left atrium, any detection of its expression would be expected to be limited to left atrium tissue samples, which severely limits the use of ccl21 as a marker for AF development.

It is amongst the objects of the present invention to obviate and/or mitigate one or more of the aforementioned disadvantages.

### Summary of the Invention

The invention is based on studies by the inventors to identify markers which may be used for the detection of AF, such as paroxysmal AF in subjects in whom access to atrial tissue is limited.

In a first aspect, there is provided a method for identifying whether or not it may be appropriate to administer to a subject a therapy for alleviating any potential consequences which may arise due to the subject having an atrial fibrillation (AF), the method comprising detecting, in a sample of fluid from the subject, a level of ddit4 expression and determining whether or not it may be appropriate to administer to the subject a therapy for alleviating any consequences which may arise due to the subject having AF, based upon the ddit4 expression level detected.

Embodiments are provided by the dependent claims.

Also provided is an assay system for use in a method as described, comprising:
a measurement device that measures ddit4 expression levels in order to provide data in relation to the level of ddit4 levels in fluid samples from a subject;
a data transformation device that acquires the ddit4 expression level data from the measurement device and performs data transformation to calculate whether or not the level determined is lower than a control, reference or normal value for ddit4 expression levels in the fluid sample; and
a database of treatment information, wherein the device identifies treatment information in the database for the level of ddit4 protein or protein fragment levels determined and outputs the treatment information to a user interface output device.

In another aspect there is provided a kit for use in a method as described, the kit comprising: at least one antibody, or probe which is/are capable of specifically binding a ddit4 protein or protein fragment thereof, wherein the antibody is labeled and/or the antibody or probe is capable of being bound to a surface, optionally wherein the antibody is labeled with a fluorescent or luminescent label.

In a related aspect, there is provided a method for identifying whether or not it may be appropriate to administer to a subject a therapy for alleviating any potential consequences which may arise due to the subject having an atrial fibrillation (AF), the method comprising detecting, in a sample of fluid from the subject, a level of ccl21 and/or ddit4 expression, and determining whether or not it may be appropriate to administer to the subject a therapy for alleviating any consequences which may arise due to the subject having AF, based upon the ccl21 and/or ddit4 expression level detected. The same method may be used, for example, to "rule out" the presence of atrial fibrillation by demonstrating high levels of ccl21 and/or ddit4.

In related embodiments, the method may further comprise treating the subject with a therapy for alleviating any consequences which may arise due to the subject having AF. The therapy may be an anticoagulant therapy but also any other therapy used in AF patients, such as rate controlling medications, antiarrhythmic drugs, or catheter ablation may be employed. Anticoagulant therapies reduce the ability of the blood to clot and hence reduce the possibility of blood clot formation which can lead to conditions such as a stroke or heart attack. Typical therapies include known anti-coagulants such as warfarin, heparin, rivaroxaban, dabigatran and apixaban, although this list is not exhaustive. Rate control medications, such as β blockers, calcium antagonists, or digitalis, slow the heart rate during AF and can therefore help the heart to work more effectively, and the patient to feel better. Antiarrhythmic drugs are medications that can prevent attacks of AF. Typical agents are amiodarone, dronedarone, flecainide, propafenone, and sotalol. Catheter ablation is an invasive procedure that can prevent AF recurrences.

Thus, in a further related aspect there is provided an anticoagulant for use in treating a subject with AF, wherein the subject has been identified as having AF based upon a level of ccl21 and/or ddit4 expression being identified in a fluid sample obtained from the subject.

Also described is a method of administering an anticoagulant drug or another AF therapy, depending on clinical need, comprising the steps of:
identifying whether or not a subject has an atrial fibrillation by detecting a reduced level of ccl21 and/or ddit4 expression in a fluid sample from the subject, in comparison to a reference level; and
administering an anticoagulant to the subject with a reduced ccl21 and/or ddit4 expression level in the fluid sample as compared to the reference level; and/or
administering a rate control therapy to a subject with a reduced ccl21 and/or ddi4 protein or protein fragment expression level in the fluid sample as compared to the reference level (depending on clinical need); and/or
administering an antiarrhythmic drug to a subject with a reduced ccl21 and/or ddi4 protein or protein fragment expression level in the fluid sample as compared to the reference level (depending on clinical need); and/or
applying catheter ablation, or a specific type of catheter ablation, to a subject with a reduced ccl21 and/or ddi4 protein or protein fragment expression level in the fluid sample as compared to the reference level (depending on clinical need).

In a further related aspect, there is provided
a method of administering an anticoagulant drug or another AF therapy, depending on clinical need, comprising the steps of:
identifying whether or not a subject has an atrial fibrillation by detecting a reduced level of ccl21 and/or ddit4 expression in a fluid sample from the subject, in comparison to a reference level; and
administering an anticoagulant to the subject with a reduced ccl21 and/or ddit4 expression level in the fluid sample as compared to the reference level; and/or
administering a rate control therapy to a subject with a reduced ccl21 and/or ddi4 protein or protein fragment expression level in the fluid sample as compared to the reference level (depending on clinical need); and/or
administering an antiarrhythmic drug to a subject with a reduced ccl21 and/or ddi4 protein or protein fragment expression level in the fluid sample as compared to the reference level (depending on clinical need); and/or
applying catheter ablation, or a specific type of catheter ablation, to a subject with a reduced ccl21 and/or ddi4 protein or protein fragment expression level in the fluid sample as compared to the reference level (depending on clinical need).

In accordance with the present invention AF may be taken to mean paroxysmal, persistent or permanent AF. The present invention is particularly suited to detecting whether or not a subject may suffer or be predisposed to suffering from paroxysmal AF and/or to the treatment of subjects with paroxysmal AF.

There is further described a method of administering an anticoagulant drug for the prevention of a blood clot which may lead to a myocardial infarction or stroke in a subject, comprising:
administering an anticoagulant drug to a subject displaying a reduced level of ccl21 and/or ddit4 expression in a fluid sample from the subject, as compared to a reference level or a predetermined threshold.

The present invention is concerned with the detection of the expression of ddit4 and optionally ccl21 in fluid samples from a subject. Preferably expression may be manifested in terms of protein or protein fragments being detected in a fluid sample, although other expression products may equally be envisaged by the skilled addressee. For the remainder of this disclosure mention will be directed to protein and/or protein fragment detection, but this should not be construed as limiting in any way, as other expression products may alternatively be detected.

Conveniently the biological sample may be any appropriate fluid sample obtained from the subject. For example, the fluid sample may comprise at least one of: urine, saliva, blood and blood fractions such as plasma, serum, sputum, semen, mucus, tears, a vaginal swab, a rectal swab, a cervical smear, a tissue biopsy, and a urethral swab. Suitably, the biological sample is one that can be readily obtained from the individual, such as urine, saliva, blood and sputum, which the individual may be able to collect from him/herself, without the need for assistance. In a preferred embodiment the biological sample is blood or a blood fraction such as plasma. It is to be understood that the present invention does not extend to non-fluid samples, such as samples of solid tissue, which may be obtained by biopsy and does not in particular extend to the sample being a sample of heart tissue.

Similarly, a fluid sample from a subject not in need of therapy and/or without AF can be obtained by any method known in the art. In order to provide a reference level of ccl21 and/or ddit4 protein or protein fragment expression, or a "normal" level of ccl21 and/or ddit4 protein or protein fragment expression determined or provided for use in determining whether or not the level of ccl21 and/or ddit4 protein or protein fragment expression in the subject being tested is substantially the same, higher or lower than "normal" or from a subject not requiring therapy and/or displaying AF.

A "reference level" for ccl21 and/or ddit4 protein or protein fragment expression as used herein, is so determined or provided in order that the subject with a ccl21 and/or ddit4 protein or protein fragment expression level lower than the predetermined value can be determined and wherein the subject is likely to be suitable and/or appropriate for anticoagulant therapy. In accordance with the present invention subjects with ccl21 and/or ddit4 protein or protein fragment expression levels lower than normal, are to be considered in accordance with the present invention as suitable and/or appropriate for treating with an anticoagulant therapy as defined herein.

"Lower" (or reduced) protein or protein fragment expression refers to decreased expression, as compared to the expression level of ccl21 and/or ddit4 protein or protein fragment in a control sample or reference level, such as from a subject or average from a population of subjects which display normal sinus rhythm and hence do not possess AF. Thus, in one aspect, the control sample or reference level is a taken/determined from a non-diseased subject or subjects, or is a published literature value for expected normal ccl21 and/or ddit4 protein or protein fragment expression. In one aspect, the differential expression, that is lower may be about 0.5 times, 1 times, 1.5 times, or alternatively, about 2.0 times, or alternatively, about 3.0 times, or alternatively, about 5 times, or alternatively, about 10 times, or alternatively about 50 times lower than the expression level in the control sample, reference level or published value. Alternatively, ccl21 and/or ddit4 protein or protein fragment expression may be referred to as "under expressed", or "down regulated".

A reduced expression level of ddit4 protein or protein fragment may be used as a basis for selecting a treatment as described herein. The expression level may be measured before and/or during treatment and the values obtained may be used by a clinician in assessing suitability and/or appropriateness for a subject to be subjected to an anticoagulant or another AF therapy. It is to be appreciated that the present invention is designed to minimise the likelihood of a subject suffering from a clot which may lead to a stroke or cardiac arrest in the future. Thus, the therapies envisaged by the present invention may be seen in general terms as prophylactic therapies as they are designed to prevent rather than cure a condition. In addition, the therapies envisaged may be selected because they are known to have specific effects in patients.

In accordance with the present invention the ddit4 and optionally ccl21 level to be determined is in relation to protein or protein fragment levels in a sample of fluid. By protein or protein fragment is meant any form of the protein or fragment thereof which may be found within a fluid sample. The term thus includes the full length wild type protein or fragments thereof, as well as mutant, allelic, splice variant and post translationally modified forms which are known in the art, or may be discovered in the future. Many suitable protein/peptide detection methods are known to the skilled addressee. Examples include radioimmunoassay (RIA), enzyme-linked immunosorbent assay (ELISA), flow cytometry, electrochemiluminescent assays, plasmon and surface enhanced resonance assays or any other sensitive quantitative method of measuring ddit4 and optionally ccl21 protein or protein fragment levels. In a preferred embodiment, the expression level is determined by an immunological method, such as a competitive or non-competitive immunoassay, preferably using a solid-phase antibody, an ELISA or ELISPOT assay.

In accordance with the invention, assay systems are provided. The assay systems include a measurement device that measures ddit4 and optionally ccl21 protein or protein fragment levels in order to provide data in relation to the level of ccl21 and/or ddit4 protein or protein fragment levels in fluid samples from a subject. The system also includes a data transformation device that acquires the ddit4 and optionally ccl21 protein or protein fragment levels data from the measurement device and performs data transformation to calculate whether or not the level determined is lower than a control, reference or normal value for ddit4 and optionally ccl21 protein or protein fragment levels in the fluid sample.

The assay system also includes an output interface device, a user interface output device to output data to a user. The assay system also includes a database of treatment information, wherein the device identifies treatment information in the database for the level of ddit4 and optionally ccl21 protein or protein fragment levels determined and outputs the treatment information to the user interface output device. In one embodiment that user interface output device may provide an output to the user, such as a subject that their ddit4 and optionally ccl21 protein or protein fragment level is lower than a control, threshold or reference value and that they should administer a suitable therapy, such as an anticoagulant therapy. In this manner a subject may continuously monitor their ddit4 and optionally ccl21 protein or protein fragment levels over a period of time and be instructed to administer a therapy only at times when their ccl21 or ddit4 protein or protein fragments levels are lower than a control, threshold or reference value. It is also possible for the output interface device to be remote from the user of the input device. For example, a subject may analyse a fluid sample at home, but the results are provided (for example wirelessly, wired or by any other suitable means) to a clinician or health care worker remote from the subject. The clinician or health care worker may then inform the subject whether or not they should administer a particular therapy based on the test results obtained.

In a further aspect there is provided a kit for use in the present methods. The kit comprises at least one antibody, or probe (e.g. a receptor or receptor fragment) which is/are capable of specifically binding the ddit4 protein or protein fragment thereof and may be labeled for example with a fluorescent or luminescent label. The kit may further comprise instructions for use, such as with an assay system as described herein.

Labels can be detected either directly, for example for fluorescent labels, or indirectly. Indirect detection can include any detection method known to one of skill in the art, including biotin-avidin interactions, antibody binding and the like. Antibodies or other ddit4 protein or protein fragment binding agents, such as a purified receptor or receptor fragment which is capable of binding to ddit4 protein or protein fragment can be bound to a surface. In one embodiment, the preferred surface is silica or glass. In another embodiment, the surface is a metal electrode.

### Detailed description of the Invention

The present invention will now be further described by way of example and with reference to the figures which show:
Figure 1: Left-right gene expression ratios of "left atrial genes" in atria of wild-type and pitx2+/- mice. All data based on mRNA expression analysis by rtPCR measured in 18 pairs of wt and pitx2+/- mice. This analysis illustrates that all genes show reduced expression in pitx2+/- atria numerically, while ccl21 and ddit4 emerge as the genes with statistically significant differences; shown are mean and SEM;
Figure 2: Reduced PITX2, ccl21, and ddit4 mRNA expression in human left and right atria with and without atrial fibrillation. Ccl21 expression is clearly reduced in atria with atrial fibrillation (striped columns, paroxysmal AF; grey columns, chronic AF) compared to those in sinus rhythm (solid black column), confirming observations of pitx2-dependent ccl21 expression in murine left atria shown in Figure 1. Shown are mean and SEM;
Figure 3: a) shows the results of a Western blot of Pitx2 levels in left and right atrial samples from patients with chronic and paroxysmal AF in comparison to patients with normal sinus rhythm; b) shows the results of a) in graphical form;
Figure 4: a) shows the results of a Western blot of ddit4 levels in left and right atrial samples from patients with chronic AF in comparison to patients with normal sinus rhythm; b) shows the results of a) in graphical form and further includes the results from patients with paroxysmal AF; and
Figure 5: shows that ccl21 plasma levels are significantly lower in left atrial and venous samples from AF patients compared to patients with other forms of cardiac arrhythmias.

### Materials and methods

For mouse, LA and RAfrom 36 mice were used (18 *Pitx2c*+/-, 18 littermate *wildtype*, age 14-20 weeks). For humans, LA and RA tissue from patients in sinus rhythm (n=7) or diagnosed with chronic (n=14) or paroxysmal AF (n=12) was used, all undergoing open heart surgery (CABG or valve surgery, median age 74). RNA was prepared using Qiagen RNeasy fibrous mini-kit and Quiagen QiaShredder Columns; cDNA was generated and RT-PCR reactions were performed using gene specific primers and SYBR Green (Life Technologies) on an ABI 7500 Fast machine.

Protein lysates were prepared using RIPA protein buffer. Samples were separated by SDS-PAGE (12% resolving/4% stacking) and blotted to membrane. Protein levels were determined by western blotting and normalized to calnexin protein expression.

Human plasma samples (both left atrial and venous blood) from patients who had undergone catheter ablation (19 with AF, 19 with other forms of cardiac arrhythmias) were used to determine CCL21 levels by ELISA (R&D Systems).

### Results

The earlier work described by Kahr et al, revealed a number of genes which showed preferred expression in left atrial tissue. Comparison of murine gene array data measured in mouse atria from wild type nad pitx2+/- mice suggested that their expression could be regulated by pitx2. However, mRNA levels were not studied in pitx2+/- mouse atria, none of the findings has been validated in human atria, and moreover as the paper highlights, even differences in mRNA concentrations do not always translate into differences in protein concentration. Hence the findings in the Kahr et al paper must be viewed with caution.

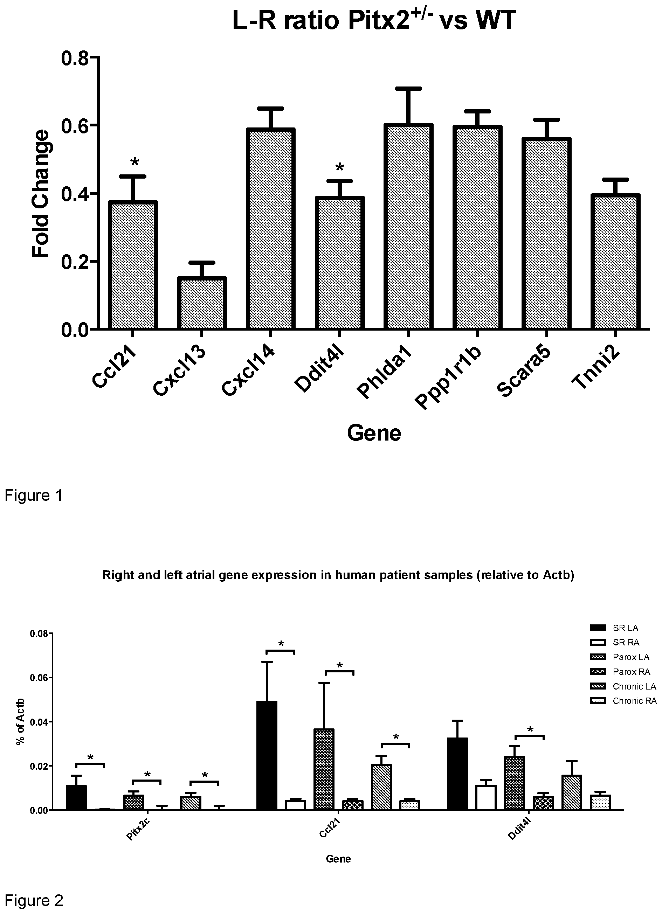

The inventors carried out rtPCR in murine atrial tissue of the 10 genes previously identified in Kahr et al as being differentially expressed between left and right atria and in comparison to pitx2 expression. The results of mouse atrial analyses are shown in Figure 1. Interesting of the 10 genes studied, only two of the genes, ddit4 and ccl21, were reduced at the mRNA level with a statistically significant difference in human left atrial tissue in AF patients compared to sinus rhythm.

Furthermore, both ccl21 and ddit4 expression was reduced in human atrial tissue collected from patients with atrial fibrillation compared to those in sinus rhythm. Patients with paroxysmal AF (i.e. with periods of AF alternating with sinus rhythm) showed less reduced ddit4 and ccl21 levels compared to patients in chronic AF (Figure 2).

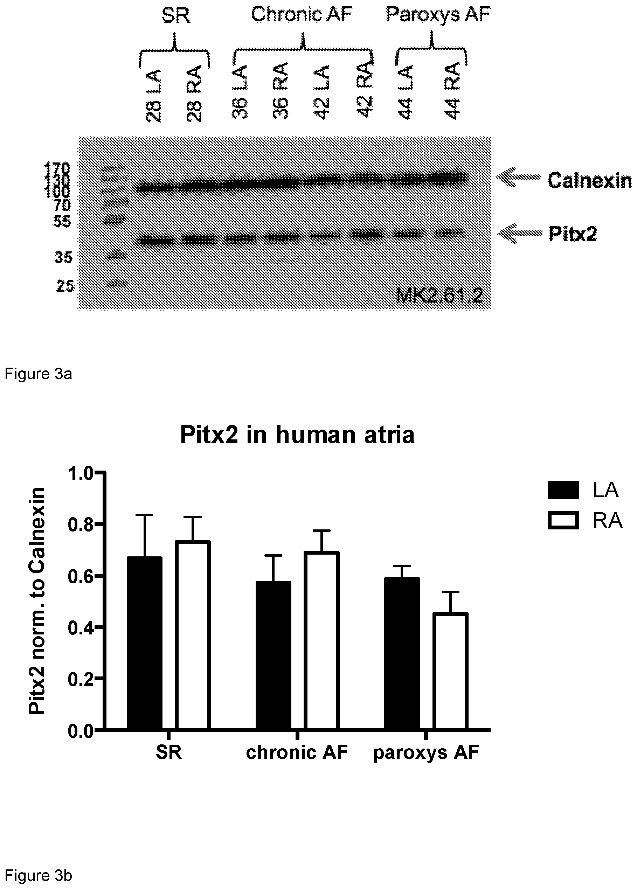

Further work showed that Pitx2c transcripts were more highly expressed in the LA compared to the RA of all patient groups (SR p=0.01; Parox p=0.00; Chronic p=0.00). Whilst there was a general trend towards down-regulation of Pitx2c transcripts in all AF patient samples compared to SR controls, none were significant (see Figure 3).

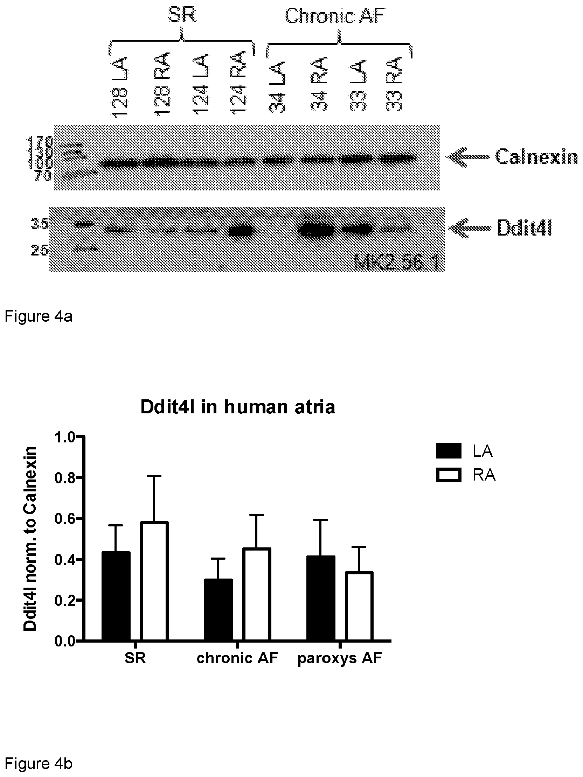

Moreover, Ccl21 was more highly expressed in the LA compared to the RA across all three patient groups (SR p=0.00; Parox p=0.03; Chronic p=0.00). Ddit4l was more highly expressed in the LA compared to the RA in patients with Paroxysmal AF (p=0.01) (see Figure 4). Furthermore, atrial Ccl21 and Ddit4l protein levels do not differ between patient groups, nor between left and right atria. However, Ccl21 was lower in RA of patients diagnosed with paroxysmal AF compared to those with chronic AF (p=0.05).

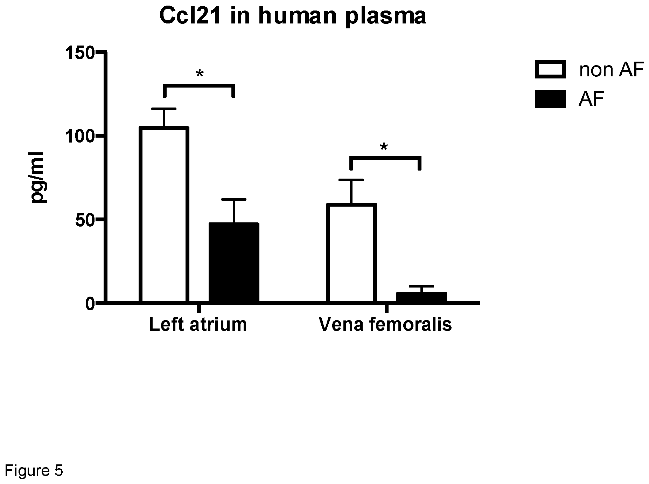

As ccl21 is a secreted chemokine, we did not expect to find a difference in ccl protein levels in atrial tissue, and we could not identify such differences in atrial tissue. Thus, we measured Ccl21 plasma levels in blood. Ccl21 levels were significantly lower in left atrial samples (p = 0.004) as well as venous samples (p = 0.002) of AF patients (n = 19) compared to patients diagnosed with other forms of cardiac arrhythmias (n = 19) (see Figure 5). Moreover, we found that in plasma venous blood ccl21 levels are markedly reduced (61.4 vs. 1.5 pg/ml) in patients with AF compared to controls (Figure 5).

In conclusion the present works shows that L-R transcript expression gradients for Ccl21 and Ddit4l are reduced in the LA of Pitx2 heterozygous mice. Pitx2, Ccl21 and Ddit4l are also differentially expressed between the L and RA of the majority of patient samples. Pitx2, Ccl21 and Ddit4l protein levels do not differ between LA and RA tissues samples of patient groups or between patient groups. However Ccl21 plasma levels are significantly lower in patients with AF compared to those with other forms of cardiac arrhythmias. The present data suggest that protein levels of Pitx2, Ccl21 and Dit4l are not reflective of transcript levels. Moreover Ccl21 and ddit4 fluid such as plasma/blood levels could be a marker for patients with atrial fibrillation.

## Claims

1. A method for identifying whether or not it may be appropriate to administer to a subject a therapy for alleviating any potential consequences which may arise due to the subject having an atrial fibrillation (AF), the method comprising detecting, in a sample of fluid from the subject, a level of ddit4 expression and determining whether or not it may be appropriate to administer to the subject a therapy for alleviating any consequences which may arise due to the subject having AF, based upon the ddit4 expression level detected, wherein the ddit4 level is reduced in comparison to a control, reference and/or threshold level and an AF therapy is indicated, or wherein the ddit4 level is elevated in comparison to a control, reference and/or threshold level and an AF therapy is not indicated, wherein the sample is a sample of urine, saliva, blood and blood fractions, serum and sputum.

2. The method according to claim 1 wherein the AF therapy is an anticoagulant therapy.

3. The method according to claim 1 wherein the AF therapy is a rate controlling medication, antiarrhythmic drugs, and/or catheter ablation.

4. The method according to any preceding claim wherein the AF is paroxysmal, persistent or permanent AF.

5. The method according to any preceding claim wherein the level of ddit4 expression is detected by an immunoassay; flow cytometry; electrochemiluminescent assay; plasmon or surface enhanced resonance assay.

6. An assay system for use in a method according to any preceding claim, comprising:
a measurement device that is configured to measure ddit4 expression levels in order to provide data in relation to the level of ddit4 levels in fluid samples from a subject;
a data transformation device that is configured to acquire the ddit4 expression level data from the measurement device and to perform data transformation to calculate whether or not the level determined is lower than a control, reference or normal value for ddit4 expression levels in the fluid sample; and
a database of treatment information, wherein the device is configured to identify treatment information according to the method of claims 1-5 in the database for the level of ddit4 protein or protein fragment or mRNA levels determined and output the treatment information to a user interface output device

7. The assay system according to claim 6 wherein the user interface output device is configured to provide an output to the user, that their ddit4 expression level is lower than a control, threshold or reference value and that the user of subject should administer a suitable therapy to the subject.

## Patentansprüche

1. Verfahren zum Identifizieren, ob es angemessen sein kann oder nicht, einer Person eine Therapie zum Abmildern möglicher Auswirkungen, die dadurch entstehen können, dass die Person ein Vorhofflimmern (AF) hat, zu verabreichen, wobei das Verfahren umfasst, in einer Probe von Flüssigkeit von der Person ein Niveau von ddit4-Expression zu detektieren und zu bestimmen, ob es angemessen sein kann oder nicht, der Person eine Therapie zum Abmildern etwaiger Auswirkungen, die dadurch entstehen können, dass die Person AF hat, zu verabreichen, basierend auf dem detektierten ddit4-Expressionsniveau, wobei das ddit4-Niveau im Vergleich mit einem Kontroll-, Referenz- und/oder Schwellenniveau reduziert ist und eine AF-Therapie indiziert ist, oder wobei das ddit4-Niveau im Vergleich mit einem Kontroll-, Referenz- und/oder Schwellenniveau erhöht ist und eine AF-Therapie nicht indiziert ist, wobei die Probe eine Probe von Urin, Speichel, Blut und Blutfraktionen, Serum und Sputum ist.

2. Verfahren nach Anspruch 1, wobei die AF-Therapie eine Antikoagulanstherapie ist.

3. Verfahren nach Anspruch 1, wobei die AF-Therapie eine Medikation zur Kontrolle der Rate, antiarrhythmische Arzneimittel und/oder eine Katheterablation ist.

4. Verfahren nach einem der vorstehenden Ansprüche, wobei das AF paroxysmales, persistentes oder permanentes AF ist.

5. Verfahren nach einem der vorstehenden Ansprüche, wobei das Niveau der ddit4-Expression durch einen Immunoassay; Durchflusszytometrie; einen elektrochemilumineszenten Assay; einen plasmon- oder oberflächenverstärkten Resonanzassay detektiert wird.

6. Assaysystem zur Verwendung in einem Verfahren nach einem der vorstehenden Ansprüche, umfassend:
eine Messvorrichtung, die konfiguriert ist, ddit4-Expressionsniveaus zu messen, um Daten in Beziehung zu dem Niveau von ddit4-Niveaus in Flüssigkeitsproben von einer Person bereitzustellen;
eine Datentransformationsvorrichtung, die konfiguriert ist, die ddit4-Expressionsniveaudaten von der Messvorrichtung zu erlangen und Datentransformation durchzuführen, um zu berechnen, ob das bestimmte Niveau niedriger ist als ein Kontroll-, Referenz- oder Normalwert für ddit4-Expressionsniveaus in der Flüssigkeitsprobe; und
eine Datenbank mit Behandlungsinformationen, wobei die Vorrichtung konfiguriert ist, Behandlungsinformationen gemäß dem Verfahren der Ansprüche 1-5 in der Datenbank für das bestimmte Niveau von ddit4-Protein oder -Proteinfragmenten oder mRNA-Niveaus zu identifizieren und die Behandlungsinformationen zu einer Benutzerschnittstellen-Ausgabevorrichtung auszugeben.

7. Assaysystem nach Anspruch 6, wobei die Benutzerschnittstellen-Ausgabevorrichtung konfiguriert ist, dem Benutzer eine Ausgabe bereitzustellen, dass sein ddit4-Expressionsniveau niedriger ist als ein Kontroll-, Schwellen- oder Referenzwert und dass der Benutzer der Person, der Person eine geeignete Therapie verabreichen sollte.

## Revendications

1. Procédé pour identifier si oui ou non il peut être approprié d'administrer à un sujet une thérapie pour atténuer de quelconques conséquences potentielles qui peuvent survenir du fait que le sujet présente une fibrillation auriculaire (AF), le procédé comprenant la détection, dans un échantillon de fluide qui est prélevé sur le sujet, d'un niveau d'expression de ddit4 et la détermination de si oui ou non il peut être approprié d'administrer au sujet une thérapie pour atténuer de quelconques conséquences qui peuvent survenir du fait que le sujet présente l'AF, sur la base du niveau d'expression de ddit4 qui a été détecté, dans lequel le niveau de ddit4 est réduit par comparaison avec un niveau de contrôle, de référence et/ou de seuil et une thérapie d'AF est indiquée, ou dans lequel le niveau de ddit4 est élevé par comparaison avec un niveau de contrôle, de référence et/ou de seuil et une thérapie d'AF n'est pas indiquée, dans lequel l'échantillon est un échantillon d'urine, de salive, de sang et de fractions constitutives du sang, de sérum et d'expectoration.

2. Procédé selon la revendication 1, dans lequel la thérapie d'AF est une thérapie par anticoagulants.

3. Procédé selon la revendication 1, dans lequel la thérapie d'AF est une médication de régulation de rythme, des médicaments antiarythmiques et/ou une ablation par cathéter.

4. Procédé selon l'une quelconque des revendications qui précèdent, dans lequel l'AF est une AF paroxysmale, persistante ou permanente.

5. Procédé selon l'une quelconque des revendications qui précèdent, dans lequel le niveau d'expression de ddit4 est détecté au moyen d'un dosage immunologique ; d'une cytométrie de flux ; d'une analyse électrochimioluminescente ; d'un essai de résonance renforcé par plasmons ou en surface.

6. Système d'analyse destiné à être utilisé au niveau d'un procédé selon l'une quelconque des revendications qui précèdent, comprenant :
un dispositif de mesure configuré pour mesurer des niveaux d'expression de ddit4 afin qu'il fournisse des données en relation avec le niveau de niveaux de ddit4 dans des échantillons de fluide qui sont prélevés sur un sujet ;
un dispositif de transformation de données configuré pour acquérir les données de niveau d'expression de ddit4 du dispositif de mesure et pour réaliser une transformation de données afin de calculer si oui ou non le niveau qui est déterminé est inférieur à une valeur de contrôle, de référence ou normale pour des niveaux d'expression de ddit4 dans l'échantillon de fluide ; et
une base de données d'informations de traitement, dans lequel le dispositif est configuré pour identifier une information de traitement conformément au procédé selon les revendications 1 à 5 dans la base de données pour le niveau de protéine ou de fragment de protéine ddit4 ou pour des niveaux de mARN qui sont déterminés et pour émettre en sortie l'information de traitement sur un dispositif de sortie d'interface utilisateur.

7. Système d'analyse selon la revendication 6, dans lequel le dispositif de sortie d'interface utilisateur est configuré pour fournir une sortie à l'utilisateur, consistant en ce que le niveau d'expression de ddit4 est inférieur à une valeur de contrôle, de seuil ou de référence et en ce que l'utilisateur en lien avec le sujet devrait administrer une thérapie appropriée au sujet.
